# EUROPEAN PATENT SPECIFICATION

(11) **EP 1 197 207 B1**
(45) Date of publication and mention of the grant of the patent: **23.04.2008**
(21) Application number: 01308284.7
(22) Date of filing: 28.09.2001
(51) Int. Cl.: A61K 9/16, A61K 31/365

(54) **Stable compositions for parenteral administration**
Stabilisierte Arzneizusammensetzungen zur parenteralen Vearbreichung
Compositions stabilisées pour l'administration parentérale

(30) Priority: 10.10.2000 US 238925 P
(43) Date of publication of application: 17.04.2002
(73) Proprietor: Wyeth Holdings Corporation, Madison, NJ 07940 (US)
(72) Inventor: Steber, William David, Ewing, New Jersey 08628 (US)
(74) Representative: Wileman, David Francis

(56) References cited:
- EP-A- 0 448 930
- EP-A- 0 525 307
- GB-A- 2 310 801
- US-A- 5 733 566

## Description

### BACKGROUND OF THE INVENTION

Helminthiasis is a widespread disease found in many animals and is responsible for significant economic losses throughout the world. Among the helminths most frequently encountered are the group of worms referred to as nematodes. The nematodes are found in the intestinal tract, heart, lungs, blood vessels and other body tissues of animals and are a primary cause of anemia, weight loss and malnutrition in the infected animals. They do serious damage to the walls and tissue of the organs in which they reside and, if left untreated, may result in death to the infected animals.

The nematodes most commonly found to be the infecting agents of ruminants include Haemonchus and Ostertagia generally found in the abomasum; Cooperia, Trichostrongylus and Nematodirus generally found in the intestinal tract, and Dictyocaulus found in the lungs. In non-ruminant animals important nematodes include Toxocara and Ancylostoma in the intestine and Dirofilaria in the heart of dogs; Ascaris in the intestine of swine; Ascaridia and Heterakis in the intestine of poultry; and large and small strongyles in equines. Treatment of animals to prevent infestation thereof by the above nematodes or to reduce or control the proliferation of these infecting agents in animals is thus an important goal.

Macromolecules such as LL-F28249α-λ compounds, 23-oxo and 23-imino derivatives of LL-F28249α-λ compounds, milbemycin compounds and avermectin compounds are useful for the prevention and treatment of infections and infestations caused by helminths, nematodes, acarids and endo- and ectoparasitic arthropods when parenterally administered to warm-blooded animals. Parenteral compositions are sterilized prior to administration to an animal. Gamma radiation is an effective sterilization process for eliminating microbial contaminants. However, certain macromolecules such as LL-F28249α-λ compounds, 23-oxo and 23-imino derivatives of LL-F28249α-λ compounds, milbemycin compounds and avermectin compounds degrade and lose much of their biological activity when irradiated. This destructive and degradative response to gamma radiation precludes the use of gamma radiation as a means to sterilize certain macromolecule-containing compositions.

Formulations for parenteral administration to warm-blooded animals containing such macromolecules have been made using antioxidants to protect the macromolecules from degradation during irradiation, for example, according to Australian Patent Number 651229 and European Patent Number 525307.

It is desirable to have formulations for parenteral administration to warm-blooded animals that will release the active macromolecule slowly over a period of time to maintain a desirable and effective level of the drug in the bloodstream of a warm-blooded animal. AU 651229 and EP 525307 teach formulations that include both a relatively hard fat and/or wax and an oil, semi-soft fat and/or fatty acid derivative which is soluble in the hard fat/wax and aids in a physical transformation of the hard fat/wax.

### SUMMARY OF THE INVENTION

The present invention relates to stable microsphere compositions which can be irradiation sterilized for parenteral administration. The compositions consist of, on a weight basis, from 50% to 99% by weight of a fat, a wax or a mixture thereof having a melting point above 40°C, t 1% to 50% of an LL-F28249α-λ compound, a 23-oxo or 23-imino derivative of an LL-F28249α-λ compound, a milbemycin compound or an avermectin compound, and 0-10% of an anti-oxidant.

The microsphere compositions of this invention do not contain an oil, semi-soft fat and/or fatty acid derivative as used in EP 525307.

Suprisingly, it has been found that the microsphere compositions of the invention can be sterilized by gamma radiation without degradation of its biological activity. Also unexpectedly, the microsphere compositions can achieve an effective sustained release effect of the water-insoluble, complex macrolides.

The invention also provides a method for introducing and maintaining blood levels of an LL-F28249α-λ compound, a 23-oxo or 23-imino derivative of an LL-F28249α-λ compound, a milbemycin compound or an avermectin compound in warm-blooded animals for an extended period of time; and a method for the prevention or treatment of infections and infestations caused by helminths, nematodes, acarids and endo- and ectoparasitic arthropods in warm-blooded animals.

### BRIEF DESCRIPTION OF THE DRAWINGS

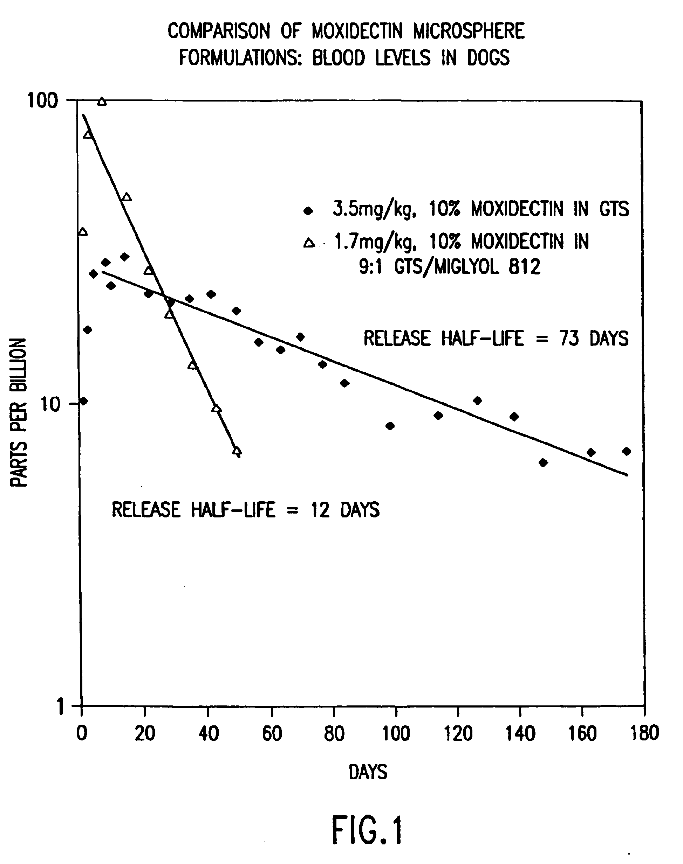

Fig. 1 illustrates and compares the release kinetics of a formulation of the invention and a formulation not within the scope of the invention.

### DETAILED DESCRIPTION OF THE INVENTION

One important aspect of the present invention is stable, slow release microsphere compositions which comprise an LL-F28249α-λ compound, a 23-oxo or 23-imino derivative of an LL-F28249α-λ compound, a milbemycin compound or an avermectin compound, dissolved in a fat, a wax or a mixture thereof having a melting point above 40°C, and preferably above 50°C. These microsphere compositions may be sterilized by gamma radiation without significant degradation. The microsphere compositions of this invention are suitable for parenteral administration by dispersion in a pharmaceutically and pharmacologically acceptable liquid vehicle.

Preferred stable microsphere compositions of the invention comprise on a weight basis 75% to 95% by weight of a fat, a wax or a mixture thereof. Preferably they contain 5% to 25% of an active ingredient selected from the group consisting of an LL-F28249α-λ compound, a 23-oxo or 23-imino derivative of an LL-F28249α-λ compound, a milbemycin compound or an avermectin compound. Preferably they contain 0.01-1% of an antioxidant.

The compounds designated LL-F28249α-λ are (collectively) isolates from the fermentation broth of the microorganism *Streptomyces cyaneogriseus* subspecies *noncyanoyenus*, deposited in the NRRL under deposit accession No. 15773. The method for preparation of LL-F28249α is disclosed in United States Patent No. 5,106,994 and its continuation, United States Patent No. 5,169,956,

The LL-F28249α-λ compounds are represented by the following structural formula: 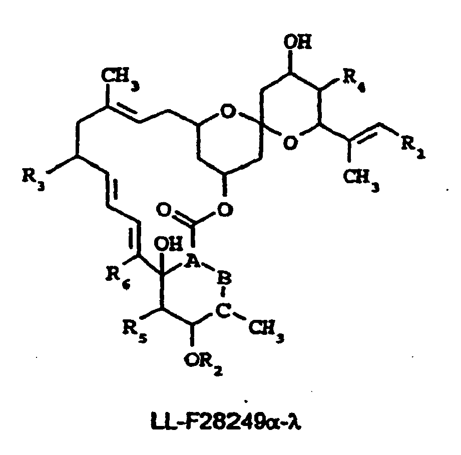 Table I, below, identifies the meanings of the variables A, B, C, and R₁₋₆ for each compound of this formula.

**TABLE I**

| **LL-F28249** | **R₁** | **R₂** | **R₃** | **R₄** | **R₅** | **R₆** | **R₅+R₆** | **A-B** | **B-C** |
|---|---|---|---|---|---|---|---|---|---|
| **alpha** | CH(CH₃)₂ | H | CH₃ | CH₃ | | | -O-CH₂- | CH-CH | CH=C |
| **beta** | CH₃ | H | CH₃ | CH₃ | | | -O-CH₂- | CH-CH | CH=C |
| **gamma** | CH₃ | CH₃ | CH₃ | CH₃ | | | -O-CH₂- | CH-CH | CH=C |
| **delta** | CH₃ | CH₃ | CH₃ | CH₃ | OH | CH₂OH | | CH-CH | CH=C |
| **epsilon** | CH(CH₃)₂ | H | H | CH₃ | | | -O-CH₂- | CH-CH | CH=C |
| **zeta** | CH₂CH₃ | H | CH₃ | CH₃ | | | -O-CH₂- | CH-CH | CH=C |
| **eta** | CH(CH₃)₂ | H | CH₃ | CH₃ | | | -O-CH₂- | C=CH | CH-CH |
| **theta** | CH(CH₃)₂ | H | CH₃ | CH₂CH₃ | | | -O-CH₂- | CH-CH | CH=C |
| **iota** | CH(CH₃)₂ | H | CH₂CH₃ | CH₃ | | | -O-CH₂- | CH-CH | CH=C |
| **kappa** | CH₃ | CH₃ | CH₃ | CH₃ | H | CH₃ | | CH-CH | CH=C |
| **lambda** | CH(CH₃)₂ | CH₃ | CH₃ | CH₃ | | | -O-CH₂- | CH-CH | CH=C |

The 23-oxo and 23-imino derivatives of LL-F28249α-λ compounds, useful in the present invention, are disclosed in U.S. Patent Number 4,916,154. Preferred compounds for use in this invention include: 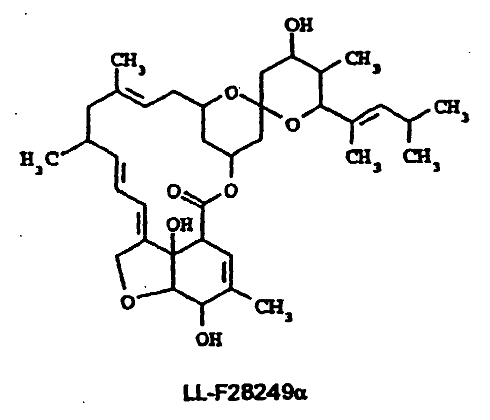 and

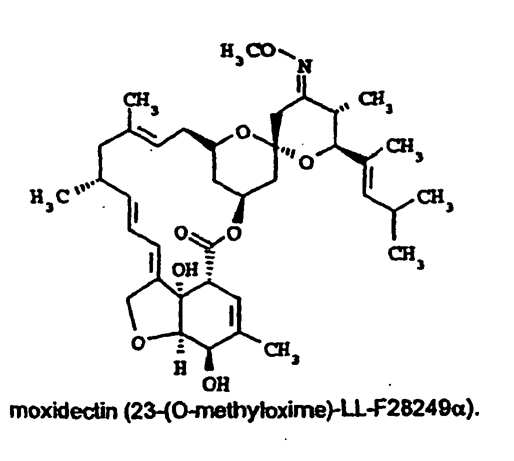

Waxes and fats which are suitable in the compositions of this invention generally have melting points higher than 40°C, preferably higher than 50°C.

The term "wax" as used herein is defined as set forth in Hawley's *The Condensed Chemical Dictionary*, Eleventh Edition, as a low-melting organic mixture or compound of high molecular weight, solid at room temperature and generally similar in composition to fats and oils except that it contains no glycerides. Some are hydrocarbons; others are esters of fatty acids and alcohols. These compounds include saturated or unsaturated long chain C₁₀-C₂₄ fatty acids, alcohols, esters, salts, ethers or mixtures thereof. They are classed among the lipids. Waxes are thermoplastic, but since they are not high polymers, they are not considered in the family of plastics. Common properties of these waxes include water repellency; smooth texture; nontoxicity; and freedom from objectionable odor and color. They are combustible and have good dielectric properties. They are soluble in most organic solvents and are insoluble in water. The major types are as follows:
I. Natural
   1. Animal (beeswax, lanolin, shellac wax, Chinese insect wax)
   2. Vegetable (camauba, candelilla, bayberry, sugar cane)
   3. Mineral (a) Fossil or earth waxes (ozocerite, ceresin, montan) (b) petroleum waxes (paraffin, microcrystalline) (slack or scale wax)
II. Synthetic
   1. Ethylenic polymers and polyol ether-esters ("Carbowax")
   2. Chlorinated naphthalenes ("Halowax")
   3. Hydrocarbon type via Fischer-Tropsch synthesis

The term "fat" as used herein is defined as set forth in Hawley's *The Condensed Chemical Dictionary,* Eleventh Edition, as a glyceryl ester of higher fatty acids such as stearic and palmitic. Such esters and their mixtures are solids at room temperatures and exhibit crystalline structure. Lard and tallow are examples. The term "fat" usually refers to triglycerides specifically, whereas "lipid" is all-inclusive.

The fat is preferably composed of triglyceryl esters of long chain C₁₂-C₂₂ fatty acids, such as stearates, palmitates, laurates, myristates, arachidates and behenates, and mixtures thereof; those having melting points greater than 50°C are most preferred. Glyceryl tristearate is a most preferred fat in the practice of this invention. Additionally, lipophilic salts of fatty acids such as magnesium stearate and the like are also suitable.

An anti-oxidant suitable in the practice of this invention includes any of the antioxidants known in the art as suitable for stabilizing the macromolecules of this invention. One preferred anti-oxidant is BHT (butylated hydroxytoluene).

The microsphere compositions of the invention may be sterilized with gamma radiation and maintain shelf life without significant loss of biological activity.

The stable microspheres of the invention are dispersed in a pharmaceutically and pharmacologically acceptable aqueous solution to obtain a slow release composition for parenteral administration.

Excipients such as surfactants, salts, buffers or mixtures thereof may be included in the vehicle of the invention. The amounts of said excipients suitable for use in the invention range from 0.1% to 20% on a weight basis. Preferably, a cellulose derivative such as hydroxypropylmethylcellulose comprises 1-5% by weight and an inorganic salt, e.g., NaCl, comprises 0.1-2% by weight of the vehicle.

Blood levels of the LL-F28249α-λ compounds, 23-oxo and 23-imino derivatives of LL-F28249α-λ compounds, milbemycin compound and avermectin compounds may be obtained and maintained for extended periods of time by injecting animals with the compositions of the invention in a suitable vehicle. Unexpectedly, the water-insoluble macrolide antibiotics can be effectively delivered from the microsphere formulation for a sustained release effect.

Maintainted blood levels of the active compounds are associated with the protection or treatment of warm-blooded animals against infections and infestation by helminths, nematodes, acarids and endo- and ectoparasitic arthropods. Maintaining the blood levels is an indication of the slow release of the active ingredient. The invention includes the use of the compositions herein to introduce and maintain levels of LL-F28249α-λ compounds, 23-oxo and 23-imino derivatives of LL-F28249α-λ compounds, milbemycin compounds and avermectin compounds in the blood stream of animals.

When parenterally administered, the compositions of this invention are highly effective for protecting or treating warm-blooded animals such as dogs, cats, cattle, sheep, horses, swine, goats, poultry and the like against infection and infestation by helminths, nematodes, acarids and endo- and ectoparasitic arthropods, including arthropod endo-parasitic infestations such as cattle grub and ecto-parasitic infestations such as psoroptic mange.

The microspheres of the invention may be prepared by dissolving the active ingredient, and optionally an anti-oxidant, in a molten fat, wax or mixture thereof and then forming microspheres of the resulting hot solution by any of the variety of techniques known in the art, such atomizing the solution. Alternatively, the solution of active ingredient and fat, waxes and mixtures thereof may be cooled to give a solid which may then be processed by procedures such as milling, grinding and the like. The microspheres, preferably fat microspheres, may be up to 1,000 µm in diameter, with a weight average size range of about 25 microns to 300 µm being preferred for parenteral administration. Preferably, the microspheres have a diameter in the approximate range of 90-180 µm.

In order to facilitate a further understanding of the invention, the following examples are presented primarily for the purpose of illustrating more specific details thereof.

### EXAMPLE 1: Preparation of 10% Moxidectin Microspheres

Glyceryl tristearate (GTS), 888.9 g., is melted in a vessel and brought to approximately 100-110°C with stirring. Moxidectin powder, 111.1 g., 90% purity and containing 0.3 - 0.4% BHT, is added to the molten GTS and stirred at 100-145°C until the powder dissolves. The hot solution is transparent and slightly yellow. This solution is transferred to an atomizing apparatus having a two-fluid nozzle or wheel atomizer, which has been preheated to about 90-110°C and set up in a spray chamber. The solution is spray atomized and microspheres of about 90-180 µm in diameter are recovered using sieves. The microspheres are filled into containers and the containers are then purged with inert gas, sealed, and gamma-irradiated to achieve sterilization.

### EXAMPLE 2: Preparation of 10% Moxidectin Microspheres

Glyceryl tristearate (GTS), 8.8 g., is melted in a vessel and brought to approximately 100-110°C with stirring. Moxidectin powder, 1.0 g., 98.9% purity and BHT, 0.002 g, is added to the molten GTS and stirred at 100-145°C until the powder dissolves. The hot solution is transparent and slightly yellow. This solution is atomized, stored and sterilized according to the procedure of Example 1.

### EXAMPLE 3: Preparation of Moxidectin Microspheres Vehicle

A vehicle containing the following ingredients is prepared as described below

| | %w/w | grams |
|---|---|---|
| NaCl | 0.9 | 90 |
| HPMC* | 2.5 | 250 |
| methylparaben | 0.18 | 18 |
| propylparaben | 0.02 | 2 |
| Water for injection, USP | q.s. | q.s. |
| Total = | 100% | 10 liters |

| | | |
|---|---|---|
| *Hydroxypropylmethylcellulose (Dow, Methocel E50, or equivalent) | | |

Approximately 50% of the water for injection is charged to a vessel and heated to about 70-80°C, and the NaCI, methylparaben and propylparaben are each added and stirred until dissolved. The HPMC is added slowly with stirring to achieve dispersion. The heat is removed and cold water for injection added to bring the volume to 10 liters. The formulation is chilled to about 5-10°C to achieve complete solubilization of the HPMC. The pH is adjusted to 4.5-5.5 by adding HCl and the solution is filtered to achieve sterilization. The vehicle solution is stored in sterile containers.

### EXAMPLE 4: Making and Using the Final Formulation

At the point of use, the vehicle made in Example 3 is added to the microspheres made in Example 1 and the container is shaken to disperse the microspheres in the vehicle. The formulation is then drawn into a syringe in a dose volume specified for the body weight of the dog to be treated and injected subcutaneously.

When a dosage of 3.5 mg of the final formulation per kg body weight is administered to dogs, the following data are collected.

| Dosage: 3.5 mg/kg, 10% moxidectin in microspheres (90-180 µm). N = 4 dogs. | |
|---|---|
| Days after dosing | Average moxidectin, ppb, in blood |
| 1 | 10.6 |
| 2 | 18.5 |
| 4 | 28.3 |
| 7 | 30.8 |
| 10 | 26.5 |
| 14 | 32.0 |
| 21 | 24.6 |
| 28 | 22.7 |
| 35 | 23.2 |
| 42 | 24.0 |
| 49 | 21.5 |
| 56 | 16.0 |
| 63 | 15.3 |
| 70 | 16.8 |
| 77 | 13.8 |
| 84 | 12.0 |
| 98 | 8.6 |
| 112 | 9.4 |
| 126 | 10.4 |
| 140 | 9.0 |
| 147 | 6.6 |
| 161 | 7.0 |
| 175 | 7.0 |

These data indicate a release half-life of 73 days. These data are shown graphically in Fig. 1.

### COMPARATIVE EXAMPLE

A formulation made according to AU 651229 and EP 525307 was administered parenterally to dogs at a dosage of 1.7 mg/kg body weight These microspheres comprised 10% moxidectin in 9:1 glyceryl tristearate/Miglyol 812 (a triglyceride oil). The results are presented in the table below and illustrated in Fig. 1.

| Dosage: 1.7 mg/kg. 10% moxidectin in microspheres (90-180 µm). N=4 dogs. | |
|---|---|
| Days after dosing | Average moxidectin, ppb, in blood |
| 1 | 38.5 |
| 3 | 80.3 |
| 8 | 100.0 |
| 15 | 50.0 |
| 22 | 28.8 |
| 29 | 21.3 |
| 35 | 14.0 |
| 42 | 9.5 |
| 49 | 6.5 |
| 56 | 5.0 |

These data indicate a release half-life of 12 days. It is apparent from the data in this Comparative Example and in Example 3 that the formulation of the present invention releases the active ingredient more gradually than does the comparative formulation. The formulation of this invention thus provides a level of active ingredient in the bloodstream that is more constant and long-lasting.

## Claims

1. A microsphere composition consisting of from 1% to 50% by weight of a compound selected from the group consisting of an LL-F28249α-λ compound, a 23-oxo or 23-imino derivative of an LL-F28249α-λ compound, a milbemycin compound and an avermectin compound; from 50% to 99% by weight of a fat, wax or mixture thereof having a melting point higher than 40°C, and 0-10%by weight of one or more anti-oxidant compounds.

2. A microsphere composition according to Claim 1 wherein said compound consists of 1% to 25% by weight of the composition.

3. A microsphere composition according to Claim 1 or Claim 2 wherein said compound is moxidectin.

4. A microsphere composition according to any one of Claims 1 to 3 in which the fat, wax or mixture thereof has a melting point higher than 50°C,

5. A microsphere composition according to any one of Claims 1 to 4 wherein said fat, wax or mixture thereof is from 75% to 95% by weight of the composition.

6. A microsphere composition according to any one of Claims 1 to 5 wherein said fat or wax is a fatty acid ester.

7. A microsphere composition according to Claim 6 wherein said fatty acid ester is a triglyceryl ester of a fatty acid having 12-22 carbon atoms.

8. A microsphere composition according to Claim 7 wherein said fatty acid ester is glyceryl tristearate.

9. A microsphere composition according to any one of Claims 1 to 8 wherein said anti-oxidant is from 0.01% to 1% by weight of the composition.

10. A microsphere composition according to Claim 1 consisting of moxidectin in an amount from 9 to 12% by weight, glyceryl tristearate in an amount from 87 to 89% by weight and the remainder is anti-oxidant.

11. A microsphere composition according to any one of Claims 1 to 10 wherein said anti-oxidant is butylated hydroxytoluene.

12. A microsphere composition according to any one of Claims 1 to 11 which has a diameter up to 1000 µm.

13. A microsphere composition according to any one of Claims 1 to 12 which has a diameter in the range from 90 to 180 µm.

14. A pharmaceutical composition suitable for injection into a warm-blooded animal comprising a microsphere composition according to any one of Claims 1 to 13 and a pharmaceutically acceptable aqueous vehicle.

15. A pharmaceutical composition according to Claim 14 wherein said aqueous vehicle comprises from 1 to 5% by weight of hydroxypropylmethylcellulose.

16. A composition for parenteral administration to warm-blooded animals comprising:
(a) microspheres consisting of 1-25% by weight of a compound selected from an LL-F28249α-λ compound, a 23-oxo or 23-imino derivative of an LL-F28249α-λ compound, a milbemycin compound and an avermectin compound; 50 to 99% by weight of a triglyceryl ester of fatty acids each having 12-22 carbon atoms, said ester having a melting point higher than 50°C and capable of dissolving said compound when in a molten state, and 0-10% by weight of one or more anti-oxidant compounds; and,
(b) a pharmaceutically acceptable aqueous vehicle.

17. A composition according to Claim 16 wherein said triglyceryl ester of fatty acids comprises glyceryl tristearate.

18. Use of a composition according to any one of Claims 1 to 17 in the preparation of a medicament for protecting or treating warm-blooded animals against infection or infestation by helminths, nematodes, acarids or endo- or ectoparasitic arthropods in a warm-blooded animal.

## Patentansprüche

1. Mikrosphärenzusammensetzung, die aus 1 Gew.-% bis 50 Gew.-% einer Verbindung, die aus der Gruppe ausgewählt ist, die aus einer LL-F28249α-λ-Verbindung, einem 23-Oxo- oder 23-Imino-Derivat einer LL-F28249α-λ-Verbindung, einer Milbemycin-Verbindung und einer Avermectin-Verbindung besteht; von 50 Gew.-% bis 99 Gew.-% eines Fetts, Wachses oder Gemischs davon mit einem Schmelzpunkt von mehr als 40 °C, und 0 - 10 Gew.-% einer oder mehrerer Antioxidationsmittelverbindungen besteht.

2. Mikrosphärenzusammensetzung nach Anspruch 1, wobei die besagte Zusammensetzung 1 Gew.-% bis 25 Gew.-% der besagten Verbindung ist.

3. Mikrosphärenzusammensetzung nach Anspruch 1 oder 2, wobei es sich bei der Verbindung um Moxidectin handelt.

4. Mikrosphärenzusammensetzung nach einem der Ansprüche 1 bis 3, in der das Fett, Wachs oder Gemisch davon einen Schmelzpunkt von mehr als 50 °C aufweist.

5. Mikrosphärenzusammensetzung nach einem der Ansprüche 1 bis 4, wobei das besagte Fett, Wachs oder Gemisch davon von 75 Gew.-% bis 95 Gew.-% der Zusammensetzung ist.

6. Mikrosphärenzusammensetzung nach einem der Ansprüche 1 bis 5, wobei es sich bei dem Fett oder Wachs um einen Fettsäureester handelt.

7. Mikrosphärenzusammensetzung nach Anspruch 6, wobei es sich bei dem Fettsäureester um einen Triglycerylester einer Fettsäure mit 12 - 22 Kohlenstoffatomen handelt.

8. Mikrosphärenzusammensetzung nach Anspruch 7, wobei es sich bei dem Fettsäureester um Glyceryltristearat handelt.

9. Mikrosphärenzusammensetzung nach einem der Ansprüche 1 bis 8, wobei das besagte Antioxidationsmittel von 0,01 Gew.-% bis 1 Gew.-% der Zusammensetzung ist.

10. Mikrosphärenzusammensetzung nach Anspruch 1, die aus Moxidectin in einer Menge von 9 bis 12 Gew.-% und Glyceryltristearat in einer Menge von 87 bis 89 Gew.-% besteht und der Rest Antioxidationsmittel ist.

11. Mikrosphärenzusammensetzung nach einem der Ansprüche 1 bis 10, wobei es sich bei dem Antioxidationsmittel um Butylhydroxytoluol handelt.

12. Mikrosphärenzusammensetzung nach einem der Ansprüche 1 bis 11, die einen Durchmesser von bis zu 1000 µm aufweist.

13. Mikrosphärenzusammensetzung nach einem der Ansprüche 1 bis 12, die einen Durchmesser im Bereich von 90 bis 180 µm aufweist.

14. Pharmazeutische Zusammensetzung, die zur Injektion in einen Warmblüter geeignet ist und eine Mikrosphärenzusammensetzung nach einem der Ansprüche 1 bis 13 und einen pharmazeutisch unbedenklichen wässrigen Träger umfasst.

15. Pharmazeutische Zusammensetzung nach Anspruch 14, wobei der wässrige Träger von 1 bis 5 Gew.-% Hydroxypropylmethylcellulose umfasst.

16. Zusammensetzung zur parenteralen Verabreichung an Warmblüter, die Folgendes umfasst:
(a) Mikrosphären, die aus 1 - 25 Gew.-% einer Verbindung, die aus einer LL-F28249α-λ-Verbindung, einem 23-Oxo- oder 23-Imino-Derivat einer LL-F28249α-λ-Verbindung, einer Milbemycin-Verbindung und einer Avermectin-Verbindung ausgewählt ist; 50 Gew.-% bis 99 Gew.-% eines Triglycerylesters von Fettsäuren mit jeweils 12 - 22 Kohlenstoffatomen, wobei der Ester einen Schmelzpunkt von mehr als 50 °C aufweist und die Verbindung in einem geschmolzenen Zustand lösen kann, und 0 - 10 Gew.-% einer oder mehrerer Antioxidationsmittelverbindungen bestehen; und
(b) einen pharmazeutisch unbedenklichen wässrigen Träger.

17. Zusammensetzung nach Anspruch 16, wobei der Triglycerylester von Fettsäuren Glyceryltristearat umfasst.

18. Verwendung einer Zusammensetzung nach einem der Ansprüche 1 bis 17 bei der Herstellung eines Arzneimittels zum Schützen oder Behandeln von Warmblütern gegen eine Infektion oder einen Befall mit Helminthen, Nematoden, Akariden oder endo- oder ektoparasitischen Arthropoden in einem Warmblüter.

## Revendications

1. Composition de microsphères constituée de 1 % à 50 % en poids d'un composé choisi parmi le groupe constitué d'un composé LL-F28249α-λ, d'un dérivé 23-oxo ou d'un dérivé 23-imino d'un composé LL-F28249 α-λ, d'un composé de milbémycine et d'un composé d'avermectine ; de 50 % à 99 % en poids d'une graisse, d'une cire, ou d'un mélange de celles-ci, ayant un point de fusion supérieur à 40 °C, et de 0 à 10 % en poids d'un ou de plusieurs composés anti-oxydants.

2. Composition de microsphères selon la revendication 1, dans laquelle ladite composition est de 1 % à 25 % en poids dudit composé .

3. Composition de microsphères selon la revendication 1 ou la revendication 2, dans laquelle ledit composé est la moxidectine.

4. Composition de microsphères selon l'une quelconque des revendications 1 à 3, dans laquelle la graisse, la cire, ou le mélange de celles-ci, a un point de fusion supérieur à 50 °C.

5. Composition de microsphères selon l'une quelconque des revendications 1 à 4, dans laquelle ladite graisse, cire, ou mélange de celles-ci est de 75 % à 95 % en poids de la composition.

6. Composition de microsphères selon l'une quelconque des revendications 1 à 5, dans laquelle ladite graisse ou ladite cire est un ester d'acide gras.

7. Composition de microsphères selon la revendication 6, dans laquelle ledit ester d'acide gras est un ester triglycérylique d'un acide gras ayant de 12 à 22 atomes de carbone.

8. Composition de microsphères selon la revendication 7, dans laquelle ledit ester d'acide gras est du tristéarate de glycéryle.

9. Composition de microsphères selon l'une quelconque des revendications 1à 8, dans laquelle ledit anti-oxydant est de 0,01 % à 1 % en poids de la composition.

10. Composition de microsphères selon la revendication 1, constituée de moxidectine dans une quantité allant de 9 à 12 % en poids, de tristéarate de glycéryle dans une quantité allant de 87 à 89 % en poids et le reste est un anti-oxydant.

11. Composition de microsphères selon l'une quelconque des revendications 1 à 10, dans laquelle ledit anti-oxydant est de l'hydroxytoluène butylé.

12. Composition de microsphères selon l'une quelconque des revendications 1 à 11, qui a un diamètre allant jusqu'à 1000 µM.

13. Composition de microsphères selon l'une quelconque des revendications 1 à 12, qui a un diamètre situé dans la plage de 90 à 180 µM.

14. Composition pharmaceutique appropriée à une injection dans un animal à sang chaud, comprenant une composition de microsphères selon l'une quelconque des revendications 1 à 13, et un véhicule aqueux pharmaceutiquement acceptable.

15. Composition pharmaceutique selon la revendication 14, dans laquelle ledit véhicule aqueux comprend de 1 à 5 % en poids d'hydroxypropylméthylcellulose.

16. Composition destinée à une administration parentérale à des animaux à sang chaud comprenant :
a) des microsphères constituées de 1 à 25 % en poids d'un composé choisi parmi un composé LL-F28249α-λ, un dérivé 23-oxo ou 23-imino d'un composé LL-F28249 α-λ, un composé de milbémycine et un composé d'avermectine ; 50% à 99% en poids d'un ester triglycérylique d'acides gras ayant chacun de 12 à 22 atomes de carbone, ledit ester ayant un point de fusion supérieur à 50 °C et étant capable de dissoudre ledit composé lorsque celui-ci est à l'état fondu, et de 0 à 10 % en poids d'un ou de plusieurs composés anti-oxydants ; et,
b) un véhicule aqueux pharmaceutiquement acceptable.

17. Composition selon la revendication 16, dans laquelle ledit ester triglycérylique d'acides gras comprend du tristéarate de glycéryle.

18. Utilisation d'une composition selon l'une quelconque des revendications 1 à 17, dans la préparation d'un médicament destiné à protéger ou à traiter des animaux à sang chaud contre une infection ou une infestation par des helminthes, des nématodes, des acariens ou des arthropodes endoparasitiques ou ectoparasitiques chez un animal à sang chaud.
